# EUROPÄISCHE PATENTSCHRIFT

(11) **EP 0 480 008 B1**
(45) Veröffentlichungstag und Bekanntmachung des Hinweises auf die Patenterteilung: **21.08.1996**
(21) Anmeldenummer: 91908718.9
(22) Anmeldetag: 24.04.1991
(51) Int. Cl.: C07C 25/18, C07C 43/225, C09K 19/30, G02F 1/137

(54) **PHENYLCYCLOHEXANE UND FLÜSSIGKRISTALLINES MEDIUM**
PHENYL CYCLOHEXANES AND LIQUID CRYSTAL MEDIUM
PHENYLCYCLOHEXANES ET MILIEU A CRISTAUX LIQUIDES

(30) Priorität: 27.04.1990 DE 4013468; 27.04.1990 DE 4013469
(43) Veröffentlichungstag der Anmeldung: 15.04.1992
(73) Patentinhaber: MERCK PATENT GmbH, D-64271 Darmstadt (DE)
(72) Erfinder: REIFFENRATH, Volker, D-6101 Rossdorf (DE); HITTICH, Reinhard, D-6101 Modautal 1 (DE); PLACH, Herbert, D-6100 Darmstadt (DE); RIEGER, Bernhard, Wacore - Tamagawagakuen, Kanagawa-pref. 227 (JP)
(86) Internationale Anmeldenummer: EP9100792
(87) Internationale Veröffentlichungsnummer: WO9117135

(56) Entgegenhaltungen:
- EP-A- 0 272 580
- DE-A- 3 139 130
- DE-A- 3 807 872
- US-A- 4 880 562

## Beschreibung

Die Erfindung betrifft neue Phenylcyclohexane der Formel I 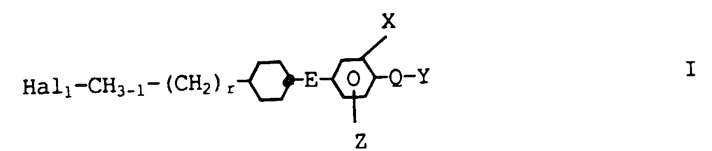 worin
Hal F oder Cl, l 1, 2 oder 3, r 1 bis 6, E -A-, -A-CH₂CH₂- oder -CH₂CH₂-A-, A trans-1,4-Cyclohexylen, 1,4-Phenylen, 3-Fluor-1,4-phenylen, 3,5-Difluor-1,4-phenylen oder eine Einfachbindung, Q -CF₂, -OCF₂, -CF₂CF₂-, -OCF₂CF₂- oder eine Einfachbindung, Y H, F oder Cl und X und Z jeweils unabhängig voneinander H oder F bedeuten.

Aus der EP-OS 0 330 216 sind Flüssigkristalle bekannt, die einen Rest der Formel F₂C=CH-(CH₂)n- (n = 0 bis 20) tragen. Die dort beschriebenen Verbindungen tragen jedoch Nitrilgruppen und stellen Flüssigkristalle mit stark positiver dielektrischer Anisotropie dar.

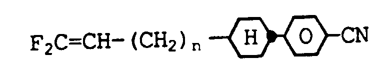

Derartige Verbindungen werden jedoch nicht den hohen Anforderungen an den elektrischen Widerstand gerecht, wie sie beispielsweise für Anzeigen mit aktiver Matrix gefordert werden. Darüberhinaus zeigen solche Verbindungen eine recht hohe Temperaturabhängigkeit der Schwellenspannung und für Benzonitrile ungewöhnlich niedrige Klärpunkte.

Die Verbindungen der Formel I können wie ähnliche, z.B. aus der DE-OS 26 36 684 bekannte Verbindungen als Komponenten flüssigkristalliner Medien verwendet werden, insbesondere für Anzeigen, die auf dem Prinzip der verdrillten Zelle beruhen.

Die bisher für diesen Zweck eingesetzten Substanzen besitzen sämtliche gewisse Nachteile, beispielsweise zu hohe Schmelzpunkte, zu niedrige Klärpunkte, zu geringe Stabilität gegenüber der Einwirkung von Wärme, Licht oder elektrischen Feldern, zu niedriger elektrischer Widerstand, ungünstige elastische Eigenschaften, zu hohe Temperaturabhängigkeit der Schwellenspannung.

Insbesondere bei Anzeigen von Supertwisttyp (STN) mit Verdrillungswinkeln von deutlich mehr als 220 °C oder bei Anzeigen mit aktiver Matrix weisen die bisher eingesetzten Materialien Nachteile auf.

Der Erfindung lag die Aufgabe zugrunde, neue flüssigkristalline Verbindungen aufzufinden, die als Komponenten flüssigkristalliner Medien geeignet sind, insbesondere für nematische Medien mit positiver dielektrischer Anisotropie, und die die Nachteile der bekannten Verbindungen nicht oder nur in geringerem Maße zeigen. Diese Aufgabe wurde durch die Bereitstellung der neuen Verbindungen der Formel 1 gelöst.

Es wurde gefunden, daß die Verbindungen der Formel I vorzüglich als Komponenten flüssigkristalliner Medien geeignet sind. Insbesondere sind mit ihrer Hilfe flüssigkristalline Medien mit weiten nematischen Bereichen, hervorragender Nematogenität bis zu tiefen Temperaturen, hervorragender chemischer Stabilität, herausragenden elastischen Eigenschaften, ausgeprägtem ε₁ bei positiver dieelektrischer Anisotropie, geringer Temperaturabhängigkeit der Schwellenspannung und/oder kleiner optischer Anisotropie erhältlich. Die neuen Verbindungen zeigen außerdem eine gute Löslichkeit für andere Komponenten derartiger Medien und hohe positive dielektrische Anisotropie bei gleichzeitig günstiger Viskosität.

Die Verbindungen der Formel I ermöglichen sowohl STN-Anzeigen mit sehr hoher Steilheit der elektrooptischen Kennlinie als auch Anzeigen mit aktiver Matrix mit hervorragender Langzeitstabilität. Durch geeignete Wahl von r und n lassen sich bei beiden Anzeigetypen die Schwellenspannungen deutlich erniedrigen.

Die Verbindungen der Formel I sind in reinem Zustand farblos und bilden flüssigkristalline Mesophasen in einem für die elektrooptische Verwendung günstig gelegenen Temperaturbereich.

Gegenstand der Erfindung sind somit die Verbindungen der Formel I sowie die Verwendung der Verbindungen der Formel I als Komponenten flüssigkristalliner Medien, flüssigkristalline Medien mit einem Gehalt an mindestens einer Verbindung der Formel I und elektrooptische anzeigen, die derartige Medien enthalten.

Vor- und nachstehend haben r, 1, E, A, X, Q, Y und Z die angegebene Bedeutung, sofern nicht ausdrücklich etwas anderes vermerkt ist.

Im Falle X = CN ist Z und/oder X vorzugsweise Fluor.

In den Verbindungen der Formel I sind die Alkylengruppen (CH₂)ᵣ vorzugsweise geradkettig. Dementsprechend bedeuten sie vorzugsweise Methylen, Ethylen, n-Propylen, n-Butylen, n-Pentylen oder n-Hexylen. r ist vorzugsweise 2 oder 4, ferner bevorzugt 3 oder 5.

Der Rest 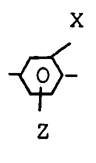 ist vorzugsweise

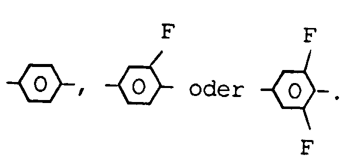

Q ist vorzugsweise -OCF₂CF₂-, -OCF₂- oder eine Einfachbindung.

Y ist vorzugsweise H oder F.

Q-Y ist vorzugsweise F, Cl, -CF₃, -OCF₃, -OCF₂H oder -OCF₂CF₂H.

Hal vorzugsweise F oder im Falle l = 1 auch Cl.

Die Verbindungen der Formel I werden im übrigen nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Zur Synthese der erfindungsgemäßen Verbindungen geeignete Vorstufen sind beispielsweise nach folgendem Syntheseschema erhältlich:

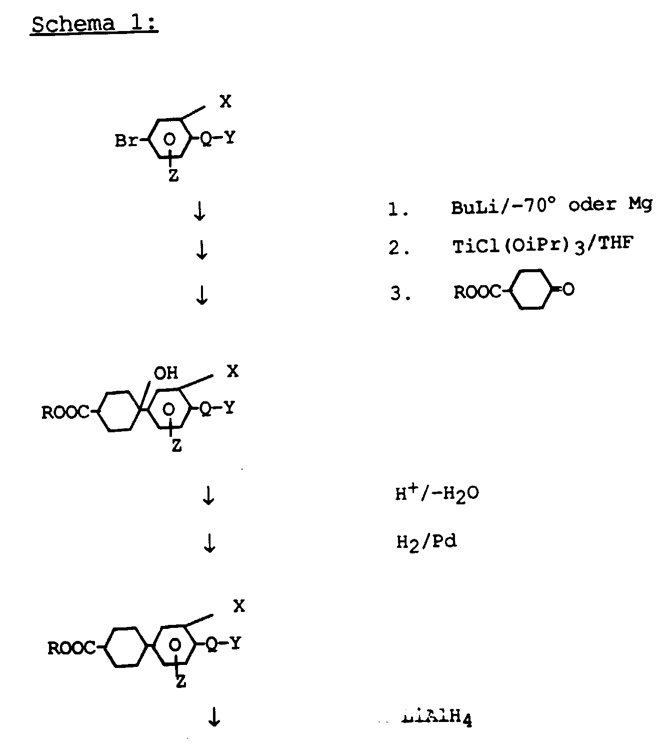

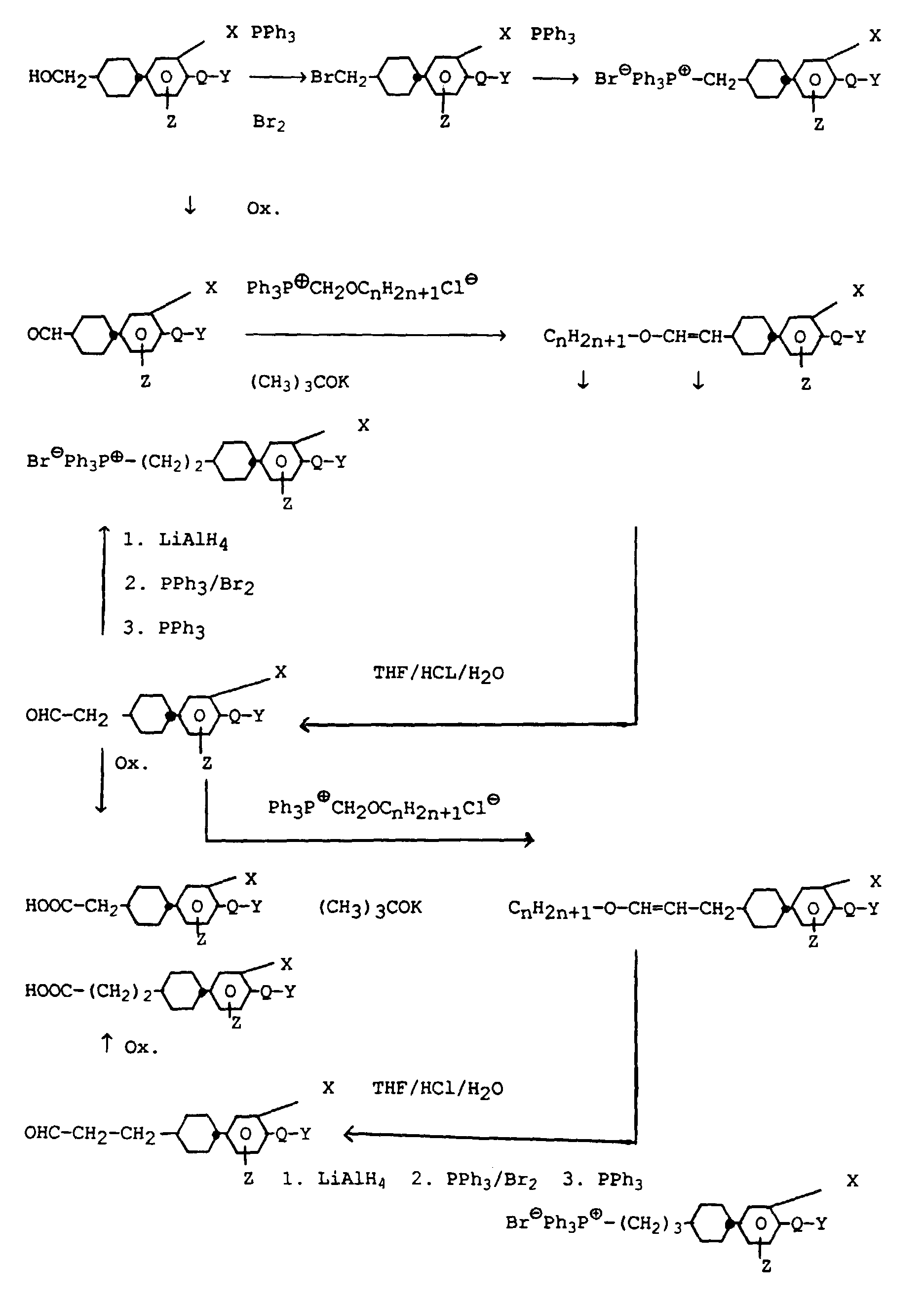

Durch sinngemäße Wiederholung dieser Reaktionssequenzen sind die höheren Homologen erhältlich.

Die aus dem entsprechenden Brombenzol-Derivat erhaltene Grignard-Verbindung wird mit Chlortrialkylorthotitanat bzw. -zirkonat nach WO 87/05599 zu dem tertiären Cyclohexanol umgesetzt. Nach Abspaltung von Wasser, Hydrierung der Doppelbindung und Isomerisierung erhält man nach üblichen Methoden den trans-Cyclohexancarbonsäureester. Aus letzterem erhält man nach üblichen Standardverfahren die geeigneten Aldehyde für die erfindungsgemäßen Verbindungen, die aus diesen durch Wittig-Synthese und nachfolgender Hydrierung der Doppelbindung erhältlich sind.

Die als Ausgangsstoffe verwendeten Brombenzolderivate sind zum Teil bekannt, zum Teil können sie ohne Schwierigkeiten nach Standardverfahren der organischen Chemie aus literaturbekannten Verbindungen hergestellt werden. Beispielsweise sind die OCF₃- oder OCHF₂-Verbindungen nach bekannten Verfahren aus den entsprechenden Phenolen bzw. die CF₃- oder CN-Verbindungen aus den entsprechenden Benzoesäuren erhältlich. Verbindungen der Formel 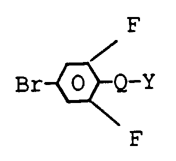 oder auch entsprechende monofluorierte Verbindungen sind beispielsweise aus den bekannten Vorstufen mit Q-Y = H durch Lithiierung bei tiefen Temperaturen und anschließende Umsetzung mit einem geeigneten Elektrophil erhältlich.

Die in obigem Reaktionsschema angegebene Homologisierung kann auch nach anderen, dem Fachmann bekannten, Standard-Verfahren erfolgen.

Die Verbindungen der Formel I, worin E 1,4-Phenylen, 3-Fluor-1,4-phenylen oder 3,5-difluor-1,4-phenylen bedeutet, sind analog obigem Syntheseschemata herstellbar, wobei anstelle des substituierten Brombenzols eine Verbindung 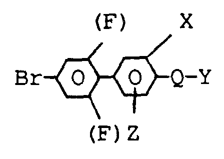 eingesetzt wird, die durch übergangsmetallkatalysierte Kopplungsreaktionen (E. Poetsch, Kontakte (Darmstadt) 1988 (2), S. 15) herstellbar ist.

Im folgenden Schema ist eine Synthese der zur Herstellung der Verbindungen mit E = trans-1,4-Cyclohexylen geeigneten Vorstufen angegeben:

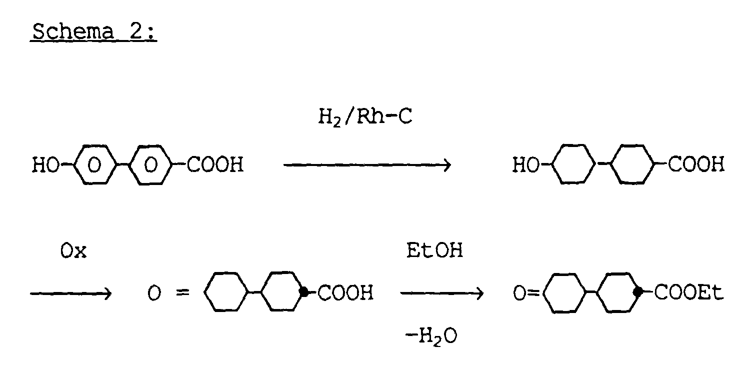

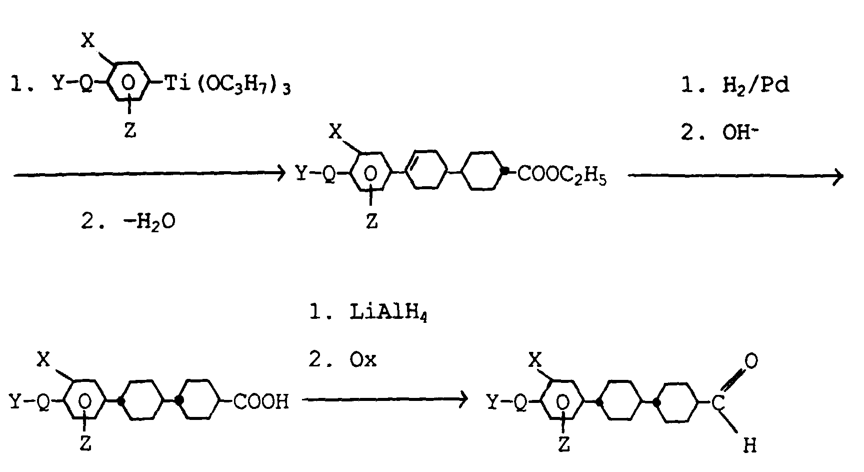

Die höheren Aldehyde, Carbonsäuren und Phosphoniumsalze können wie in Schema 1 angegeben hergestellt werden. Es ist für den Fachmann offensichtlich, daß nach Schema 2 durch Dehydrierung der Cyclohexenzwischenstufe (z.B. mit Chloranil) in analoger Weise auch die Verbindungen mit E = 1,4-Phenylen zugänglich sind.

Die Verbindungen der Formel I können nun nach an sich bekannten Methoden durch Umsetzungen von ω-Fluor-, ω-Chlor-, ω-Di- oder ω-Tri-fluor- bzw. ω-Di- oder ω-Trichlor-n-alkanalen mit den oben beschriebenen Phosphoniumsalzen der Formel 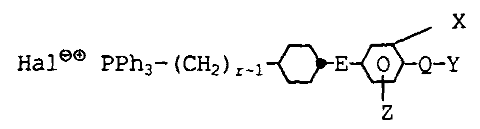 nach Wittig und nachfolgender Hydrierung erhalten werden, wobei die Art des ω-Halogenalkanals und r je nach gewünschtem Zielprodukt gewählt werden.

Es ist jedoch auch möglich, umgekehrt die oben beschriebenen Aldehyde mit Phosphoniumsalzen von ω-Fluor-, ω-Chlor-, ω-Di- oder ω-Trifluoralkylhalogeniden nach Wittig umzusetzen. Verbindungen der Formel I sind ebenfalls leicht zugänglich durch Umsetzung der oben beschriebenen Aldehyde mit Fluorierungsmitteln wie DAST oder durch Umsetzung der beschriebenen Carbonsäuren mit SF₄. Ganz besonders bevorzugte Synthesevarianten sind den folgenden Schemata zu entnehmen:

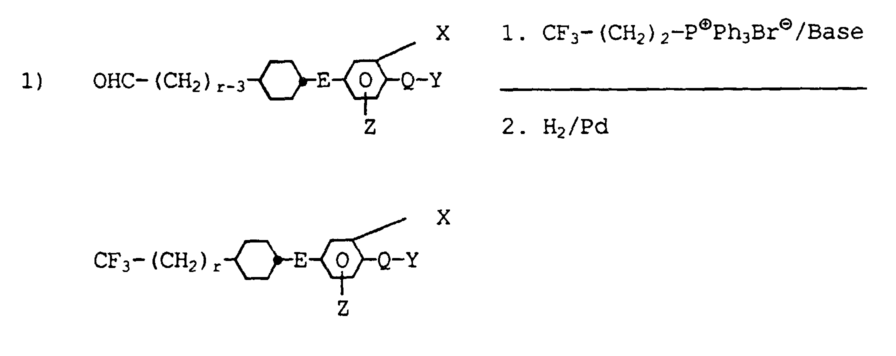

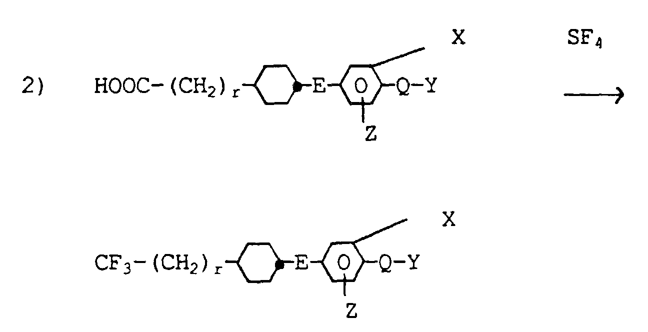

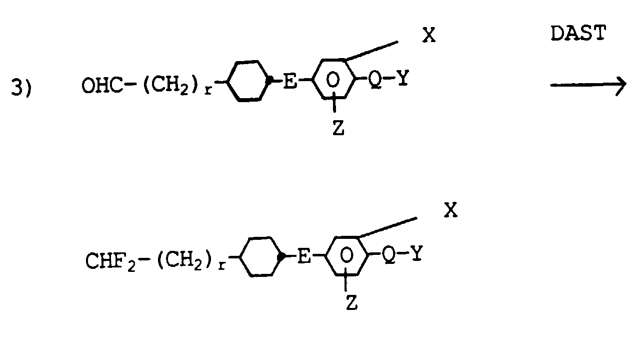

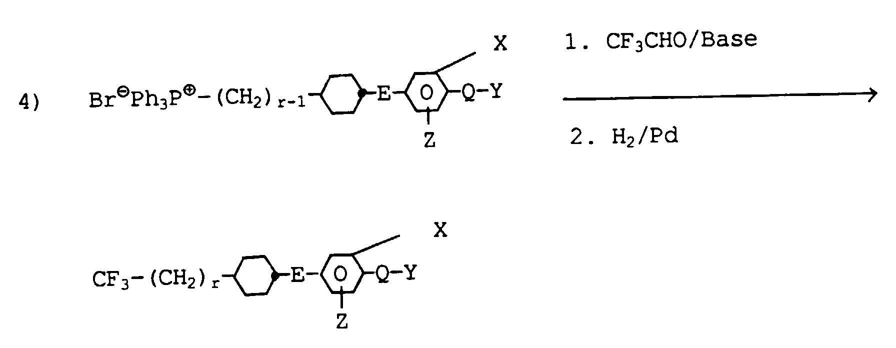

Entsprechende Ausgangsmaterialien wie CF₃-(CH₂)ₓ-CHO und CF₃-(CH₂)ₓ-Hal und CHF₂-(CH₂)ₓ-Hal (Hal = Br oder J/x = 1, 2 oder 3) sind dem Fachmann bekannt und in der Literatur beschrieben.

Verbindungen der Formel I mit l = 1 sind ebenfalls leicht zugänglich durch Umsetzung der oben beschriebenen Aldehyde mit Halogenierungsmitteln wie Thionylchlorid oder Tetraalkylammoniumfluorid. Ganz besonders bevorzugte Synthesevarianten sind den folgenden Schemata zu entnehmen:

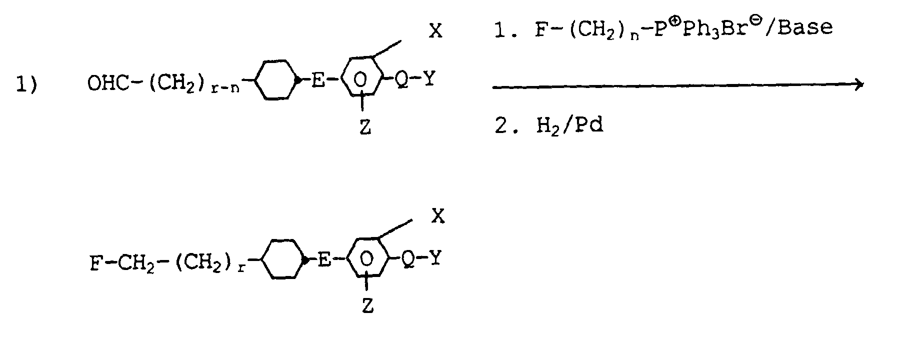

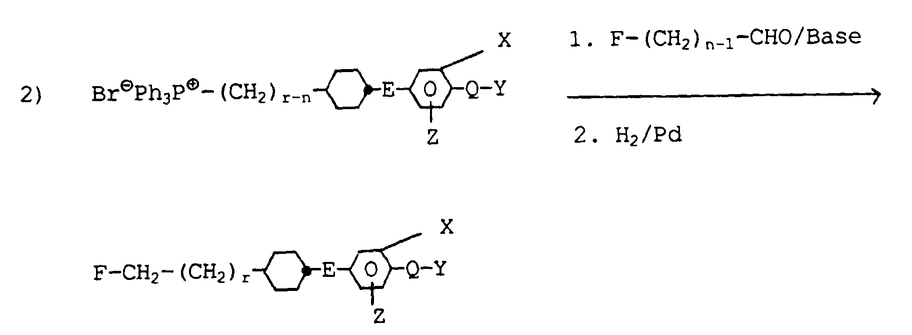

Entsprechende Ausgangsmaterialien wie Hal-(CH₂)ₓ-CHO und F-(CH₂)ₓ-Hal (Hal = Br oder J/x = 1, 2 oder 3) sind dem Fachmann bekannt und in der Literatur beschrieben.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Biphenyle, Terphenyle, Phenyl- oder Cyclohexylbenzoate, Cyclohexancarbonsäurephenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclo∼ hexylpyridine, Phenyl- oder Cyclohexyldioxane, Phenyl- oder Cyclohexyl-1,3-dithiane, 1,2-Diphenylethane, 1,2-Dicyclo hexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane und Tolane.

Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R' -L-E-R" 1

R' -L-COO-E-R" 2

R' -L-OOC-E-R" 3

R'-L-CH₂CH₂-E-R" 4

R'-L-CC-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in den Verbindungen der Teil formeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R" voneinander verschieden, wobei einer dieser Reste meist Alkyl oder Alkenyl ist. In den Verbindungen der Teilformeln 1b, 2b, 3b, 4b und 5b bedeutet R" -CN, -CF₃, -OCF₃, F, Cl oder -NCS; R hat dabei die bei den Verbindungen der Teilformeln la bis 5a angegebene Bedeutung und ist vorzugsweise Alkyl oder Alkenyl. Besonders bevorzugt ist R" ausgewählt aus der Gruppe bestehend aus -F, Cl, CF₃ und -OCF₃. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. mp. = Schmelzpunkt, cp. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

Es bedeuten ferner:

K: Kristallin-fester Zustand, S: smektische Phase (der Index kennzeichnet den Phasentyp), N: nematischer Zustand, Ch: cholesterische Phase, I: isotrope Phase. Die zwischen zwei Symbolen stehende Zahl gibt die Umwandlungstemperatur in Grad Celsius an.
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- DMSO: Dimethylsulfoxid
- KOT: Kalium-tertiär-butanolat
- THF: Tetrahydrofuran
- pTSOH: p-Toluolsulfonsäure

Der Substituent Z ist in den folgenden Beispielen jeweils in ortho-Position zu Q-Y.

### Beispiel 1

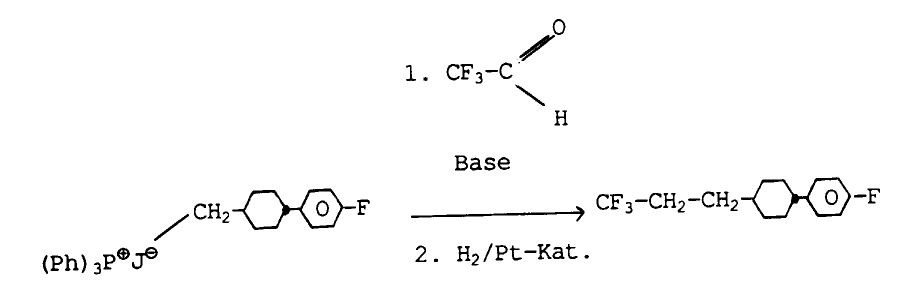

Zu einer Suspension von 0,2 m Phosphoniumsalz in 500 ml THF tropft man bei 0-5 °C eine Lösung von Kalium-tert.-butylat in THF. Es entsteht eine organge-gefärbte Ylid-Suspension, in die bei 0-5 °C Trifluoracetaldehydgas, bis zur Entfärbung, eingeleitet wird. Nach extraktiver Aufbereitung und Filtration über Kieselgel erhält man das Alken (cis-trans-Gemisch), welches in Ethanol gelöst, nach Zusatz von PtO₂-Katalysator, zum Produkt hydriert wird.

Völlig analog bzw. nach obigem Syntheseschemata erhält man die folgenden Verbindungen der Formel I
(E = Einfachbindung, Hal = F, 1 = 3):

| r | Q-Y | X | z |
|---|---|---|---|
| 3 | F | H | H |
| 4 | F | H | H |
| 5 | F | H | H |
| 6 | F | H | H |
| 2 | F | F | H |
| 4 | F | F | H |
| 2 | Cl | H | H |
| 4 | Cl | H | H |
| 2 | Cl | F | F |
| 4 | Cl | F | F |
| 2 | CF₃ | H | H |
| 4 | CF₃ | H | H |
| 2 | OCF₃ | H | H |
| 4 | OCF₃ | H | H |
| 2 | OCHF₂ | H | H |
| 4 | OCHF₂ | H | H |
| 2 | OCHF₂ | F | H |
| 4 | OCHF₂ | F | H |

sowie die Verbindungen der Formel I mit E = Einfachbindung, Hal = F und l = 2:

| r | Q-Y | X | z |
|---|---|---|---|
| 3 | F | H | H |
| 4 | F | H | H |
| 5 | F | H | H |
| 6 | F | H | H |
| 2 | F | F | H |
| 4 | F | F | H |
| 2 | Cl | H | H |
| 4 | Cl | H | H |
| 2 | Cl | F | F |
| 4 | Cl | F | F |
| 2 | CF₃ | H | H |
| 4 | CF₃ | H | H |
| 2 | OCF₃ | H | H |
| 4 | OCF₃ | H | H |
| 2 | OCHF₂ | H | H |
| 4 | OCHF₂ | H | H |
| 2 | OCHF₂ | F | H |
| 4 | OCHF₂ | F | H |

### Beispiel 2

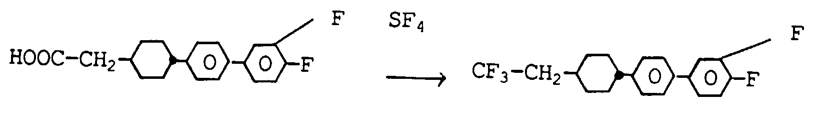

0,1 m der Carbonsäure werden mit 0,4 m SF₄ in einem Autoklaven 10 h auf 130 °C erhitzt. Aus dem Rohgemisch erhält man das Produkt durch extraktive Aufarbeitung mit anschließender chromatographischer Aufreinigung und Kristallisation.

Völlig analog bzw. nach obigen Syntheseschemata erhält man die folgenden Verbindungen der Formel I
(E = 1,4-Phenylen, Hal = F, l = 3):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | H |
| 2 | Cl | F | H |
| 4 | Cl | F | H |

| r | Q-Y | X | z |
|---|---|---|---|
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |

sowie die folgenden Verbindungen der Formel I
(E = 3-Fluor-1,4-Phenylen, Hal = F, l = 3):

| r | Q-Y | X | z |
|---|---|---|---|
| 2 | F | F | H |
| 3 | F | F | H |
| 4 | F | F | H |
| 1 | F | F | F |
| 2 | F | F | F |
| 4 | F | F | F |
| 1 | Cl | H | H |
| 2 | Cl | H | H |
| 4 | Cl | H | H |
| 1 | Cl | F | H |
| 2 | Cl | F | H |
| 4 | Cl | F | H |

| r | Q-Y | X | z |
|---|---|---|---|
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = 1,4-Phenylen, Hal = F, l = 2):

| r | Q-Y | X | z |
|---|---|---|---|
| 2 | F | F | H |
| 3 | F | F | H |
| 4 | F | F | H |
| 1 | F | F | F |
| 2 | F | F | F |
| 4 | F | F | F |
| 1 | Cl | H | H |
| 2 | Cl | H | H |
| 4 | Cl | H | H |

| r | Q-Y | X | z |
|---|---|---|---|
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = 3,5-Difluor-1,4-Phenylen, Hal = F, l = 2):

| r | Q-Y | X | z |
|---|---|---|---|
| 2 | F | F | H |
| 3 | F | F | H |
| 4 | F | F | H |
| 1 | F | H | H |
| 2 | F | H | H |
| 4 | F | H | H |

| r | Q-Y | X | z |
|---|---|---|---|
| 1 | Cl | H | H |
| 2 | Cl | H | H |
| 4 | Cl | H | H |
| 1 | Cl | F | H |
| 2 | Cl | F | H |
| 4 | Cl | F | H |
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

### Beispiel 3

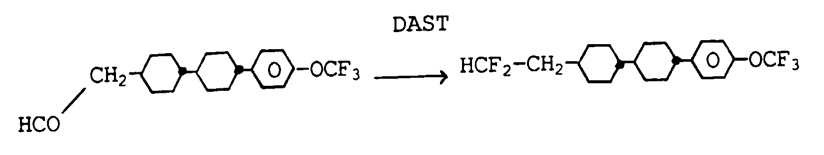

0,02 m des Aldehyds werden in 50 ml THF gelöst und mit 0,05 m DAST versetzt. Man rührt noch 24 h bei RT und isoliert das Produkt durch extraktive Aufarbeitung mit nachfolgender chromatographischer Aufreinigung.

Völlig analog bzw. nach obigen Syntheseschemata erhält man die folgenden Verbindungen der Formel I
(E = trans-1,4-Cyclohexylen, Hal = F, l = 2):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | H |
| 2 | Cl | F | H |
| 4 | Cl | F | H |

| r | Q-Y | X | z |
|---|---|---|---|
| 1 | CF₃ | H | H |
| 2 | CF₃ | H | H |
| 4 | CF₃ | H | H |
| 1 | OCF₃ | H | H |
| 3 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = trans-1,4-Cyclohexylen, Hal = F, l = 3):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |

| r | Q-Y | X | z |
|---|---|---|---|
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

### Beispiel 4

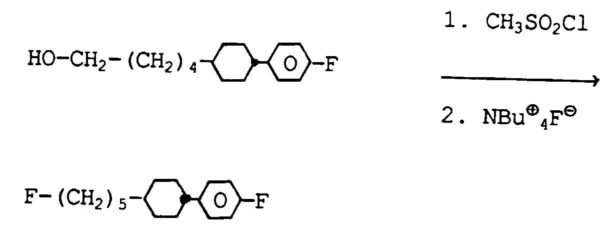

Ein Gemisch von 0,1 m des Mesylats und 0,3 m Tetrabutylammoniumfluorid in 300 ml Acetonitril wird 4 Stunden am Rückfluß gekocht. Nach Eindampfen und extraktiver Aufarbeitung wird das Rohprodukt durch Chromatographie aufgereinigt

Völlig analog bzw. nach obigem Syntheseschemata erhält man die folgenden Verbindungen der Formel I
(E = Einfachbindung, l = 1, Hal = F) :

| r | Q-Y | X | z |
|---|---|---|---|
| 3 | F | H | H |
| 5 | F | H | H |
| 6 | F | H | H |
| 7 | F | H | H |
| 2 | F | F | H |
| 4 | F | F | H |
| 2 | Cl | H | H |
| 4 | Cl | H | H |
| 2 | Cl | F | F |
| 4 | Cl | F | F |

| r | Q-Y | X | z |
|---|---|---|---|
| 2 | CF₃ | H | H |
| 4 | CF₃ | H | H |
| 2 | OCF₃ | H | H |
| 4 | OCF₃ | H | H |
| 2 | OCHF₂ | H | H |
| 4 | OCHF₂ | H | H |
| 2 | OCHF₂ | F | H |
| 4 | OCHF₂ | F | H |

sowie die Verbindungen der Formel I mit E = Einfachbindung, l = 1 und Hal = Cl:

| r | Q-Y | X | z |
|---|---|---|---|
| 3 | F | H | H |
| 5 | F | H | H |
| 6 | F | H | H |
| 7 | F | H | H |
| 2 | F | F | H |
| 4 | F | F | H |
| 2 | Cl | H | H |
| 4 | Cl | H | H |
| 2 | Cl | F | F |
| 4 | Cl | F | F |

| r | Q-Y | X | z |
|---|---|---|---|
| 2 | CF₃ | H | H |
| 4 | CF₃ | H | H |
| 2 | OCF₃ | H | H |
| 4 | OCF₃ | H | H |
| 2 | OCHF₂ | H | H |
| 4 | OCHF₂ | H | H |
| 2 | OCHF₂ | F | H |
| 4 | OCHF₂ | F | H |

### Beispiel 5

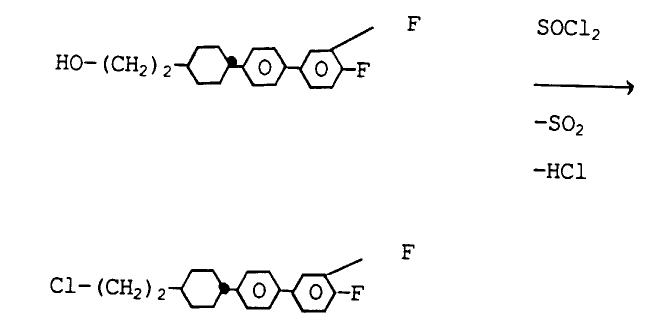

Ein Gemisch aus 0,05 m des Alkohols und 0,1 m Thionylchlori wird 6 Stunden am Rückfluß gekocht und wie in Beispiel 4 aufgearbeitet.

Völlig analog bzw. nach obigen Syntheseschemata erhält man die folgenden Verbindungen der Formel I
(E = 1,4-Phenylen, l = 1, Hal = Cl):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |

| r | Q-Y | X | z |
|---|---|---|---|
| 1 | OCHF₂ | F | H |
| 2 | OCHF₂ | F | H |
| 4 | OCHF₂ | F | H |

sowie die folgenden Verbindungen der Formel I
(E = 3-Fluor-1,4-Phenylen, l = 1, Hal = Cl):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | F | H |
| 2 | OCF₃ | F | H |
| 4 | OCF₃ | F | H |

| r | Q-Y | X | z |
|---|---|---|---|
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = 1,4-Phenylen, l = 1, Hal = F):

| r | Q-Y | X | z |
|---|---|---|---|
| 2 | F | F | H |
| 3 | F | F | H |
| 4 | F | F | H |
| 1 | F | F | F, K 35 I |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |

| r | Q-Y | X | z |
|---|---|---|---|
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = 3-Fluor-1,4-Phenylen, l = 1, Hal = F) :

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |

| r | Q-Y | X | z |
|---|---|---|---|
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

sowie die folgenden Verbindungen der Formel I
(E = 3,5-Difluor-1,4-Phenylen, l = 1, Hal = F):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |

| r | Q-Y | X | z |
|---|---|---|---|
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | H | H |
| 2 | OCF₃ | H | H |
| 4 | OCF₃ | H | H |
| 1 | OCHF₂ | H | H |
| 2 | OCHF₂ | H | H |
| 4 | OCHF₂ | H | H |
| 1 | OCHF₂ | F | H |
| 2 | OCHF₂ | F | H |
| 4 | OCHF₂ | F | H |

### Beispiel 6

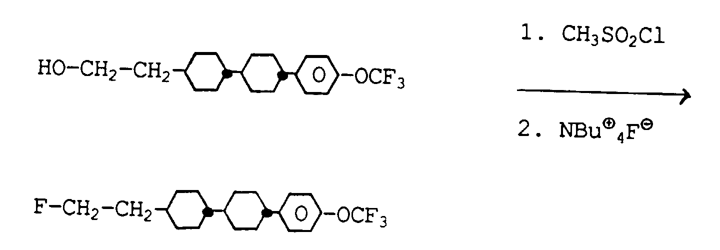

Analog Beispiel 4 erhält man das Zielprodukt durch Umsetzung des Mesylats mit Tetrabutylammoniumfluorid.

Völlig analog bzw. nach obigen Syntheseschemata erhält man die folgenden Verbindungen der Formel I
(E = trans-1,4-Cyclohexylen, l = 1, Hal = F) :

| r | Q-Y | X | z |
|---|---|---|---|
| 2 | F | F | H |
| 3 | F | F | H |
| 4 | F | F | H |
| 1 | F | F | F, K 64 I |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 2 | OCF₃ | F | H |
| 3 | OCF₃ | F | H |
| 4 | OCF₃ | F | H |
| 1 | OCHF₂ | H | H |
| 2 | OCHF₂ | H | H |
| 4 | OCHF₂ | H | H |

| r | Q-Y | X | z |
|---|---|---|---|
| 1 | OCHF₂ | F | H |
| 2 | OCHF₂ | F | H |
| 4 | OCHF₂ | F | H |

sowie die folgenden Verbindungen der Formel I
(E = trans-1,4-Cyclohexylen, l = 1, Hal = Cl):

| r | Q-Y | X | z |
|---|---|---|---|
| 2 | F | F | H |
| 3 | F | F | H |
| 4 | F | F | H |
| 1 | F | F | F |
| 2 | F | F | F |
| 3 | F | F | F |
| 4 | F | F | F |
| 1 | Cl | H | H |
| 2 | Cl | H | H |
| 4 | Cl | H | H |
| 1 | Cl | F | F |
| 2 | Cl | F | F |
| 4 | Cl | F | F |
| 1 | CF₃ | H | H |
| 2 | CF₃ | H | H |
| 4 | CF₃ | H | H |
| 1 | OCF₃ | F | H |
| 2 | OCF₃ | F | H |
| 4 | OCF₃ | F | H |

| r | Q-Y | X | z |
|---|---|---|---|
| 1 | OCHF₂ | F | H |
| 2 | OCHF₂ | F | H |
| 4 | OCHF₂ | F | H |
| 1 | OCHF₂ | F | F |
| 2 | OCHF₂ | F | F |
| 4 | OCHF₂ | F | F |

## Patentansprüche

1. Phenylcyclohexane der Formel I 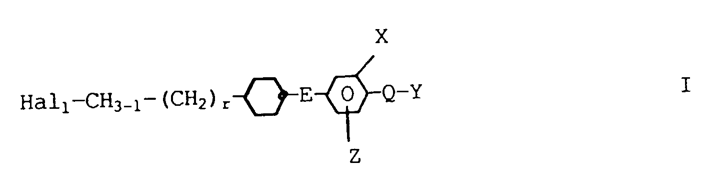 worin
Hal F oder Cl, l 1, 2 oder 3, r 1 bis 6, E -A-, -A-CH₂CH₂- oder -CH₂CH₂-A-, A trans-1,4-Cyclohexylen, 1,4-Phenylen, 3-Fluor-1,4-phenylen, 3,5-Difluor-1,4-phenylen oder eine Einfachbindung, Q -CF₂, -OCF₂, -CF₂CF₂-, -OCF₂CF₂- oder eine Einfachbindung, Y H, F oder Cl und X und Z jeweils unabhängig voneinander H oder F bedeuten.

2. Verwendung der Phenylcyclohexane der Formel I nach Anspruch 1 als Komponenten flüssigkristalliner Medien für elektrooptische Anzeigen.

3. Flüssigkristallines Medium für elektrooptische Anzeigen mit mindestens zwei flüssigkristallinen Komponenten, dadurch gekennzeichnet, daß mindestens eine Komponente ein Phenylcyclohexan der Formel I nach Anspruch 1 ist.

4. Elektrooptische Anzeige auf der Basis einer Flüssigkristallzelle, dadurch gekennzeichnet, daß die Flüssigkristallzelle ein Medium nach Anspruch 3 enthält.

## Claims

1. Phenylcyclohexanes of the formula I 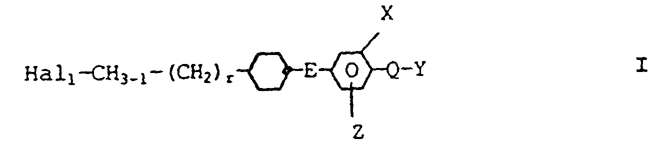 in which
Hal is F or Cl, l is 1, 2 or 3, r is 1 to 6, E is -A-, -A-CH₂CH₂- or -CH₂CH₂-A-, A is trans-1,4-cyclohexylene, 1,4-phenylene, 3-fluoro-1,4-phenylene, 3,5-difluoro-1,4-phenylene or a single bond, Q is -CF₂, -OCF₂, -CF₂CF₂-, -OCF₂CF₂- or a single bond, Y is H, F or Cl and X and Z are each, independently of one another, H or F.

2. Use of the phenylcyclohexanes of the formula I according to claim 1 as components of liquid-crystalline media for electrooptical displays.

3. Liquid-crystalline medium for electrooptical displays having at least two liquid-crystalline components, characterized in that at least one component is a phenylcyclohexane of the formula I according to claim 1.

4. Electrooptical display based on a liquid-crystal cell, characterized in that the liquid-crystal cell contains a medium according to claim 3.

## Revendications

1. Phenylcyclohexanes de formule I 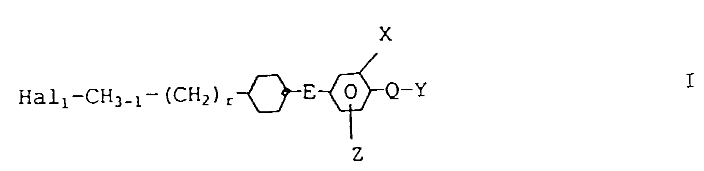 dans laquelle
Hal est F ou Cl, l vaut 1, 2 ou 3, r vaut de 1 à 6, E est -A-, -A-CH₂CH₂- ou -CH₂CH₂-A-, A est le radical trans-1,4-cyclohexylène, 1,4-phénylène, 3-fluoro-1,4-phénylène, 3,5-difluoro-1,4-phénylène ou une liaison simple, Q est -CF₂, -OCF₂, -CF₂CF₂-, -OCF₂CF₂- ou une liaison simple, Y est H, F ou Cl, et X et Z sont chacun, indépendamment l'un de l'autre, H ou F.

2. Utilisation des phénylcyclohexènes de formule I selon la revendication 1 comme constituants de milieux à cristaux liquides pour dispositifs d'affichage électro-optiques.

3. Milieu à cristaux liquides pour dispositifs d'affichage électro-optiques ayant au moins deux constituants mésomorphes, caractérisé en ce qu'un constituant est un phénylcyclohexane de formule I selon la revendication 1.

4. Dispositif d'affichage électro-optique à base d'une cellule à cristaux liquides, caractérisé en ce que la cellule à cristaux liquides contient un milieu selon la revendication 3.
